# EUROPEAN PATENT APPLICATION

(11) **EP 1 655 000 A1**
(43) Date of publication of application: **10.05.2006**
(21) Application number: 04747736.9
(22) Date of filing: 13.07.2004
(51) Int. Cl.: A61C 17/00, A61C 19/00, A61B 1/24, A61B 1/06

(54) **MEDICAL LIGHT EMITTING DEVICE**

(30) Priority: 14.07.2003 JP 2003196447
(71) Applicant: Showa Yakuhin Kako Co., Ltd, Tokyo 104-0031 (JP)
(72) Inventor: SAKAGUCHI, Hidenari, Showa Yakuhin Kako Co., Ltd., Chuo-ku, Tokyo 1040031 (JP)
(74) Representative: Sternagel, Fleischer, Godemeyer & Partner
(86) International application number: PCT/JP2004/010272
(87) International publication number: WO 2005/004745

(57) **Abstract**

It is an object of the present invention to provide a means which can increase the residence property at an affected site of a dental therapeutic agent such as a therapeutic agent for periodontal disease when such agent is applied to the affected site, and also can suppress the diffusion of the agent. The present invention provides a light-emitting device for medical use that is composed of resin comprising illuminant or fluorescence substance.

## Description

### Technical Field

The present invention relates to a light-emitting device for medical use and particularly relates to a light-emitting device for internal use such as a light-emitting device for the oral cavity using a mouthpiece comprising illuminant or fluorescence substance.

### Background Art

In general, a mouthpiece, a mouthguard, or what can be referred to as a "night form preguard has been used for protection of the oral cavity during dental treatment or hard exercise such as boxing, prevention of bruxism during sleep, and the like. In addition, JP Patent Publication (Kokai) No. 6-217996 (1994) discloses a method of forming a mouthguard for toothbrushing, to the inside surface of which toothpaste is applied, which is characterized in that plasticity is imparted via heating to a mouthguard for toothbrushing made of thermoplastic resin, a groove is formed on the mouthguard so as to correspond to a tooth form, and toothpaste is applied inside the groove, such that the mouthguard can impart toothpaste to the gum.

Meanwhile, improved esthetics relating to teeth have been strongly desired in dental treatment, and thus teeth bleaching has been performed. Tooth discoloration or staining occurs due to deposition of chromatic substances contained in tobaccos or favorite foods such as coffee and multiplication of pigment-producing bacteria. Also, tooth discoloration occurs due to external factors such as influences of metallic materials in the oral cavity, or the like. In addition, tooth discoloration occurs due to internal factors such as aging, metabolic disorders, inheritance, and dental disorders. To cope with such discoloration derived from external or internal factors, a method for bleaching teeth using a peroxide such as hydrogen peroxide or urea peroxide, a reductant, an acid, or an alkali has been employed. To promote bleaching effects obtained by such agents, heating or light irradiation may be carried out in combination. Further, light irradiation is often performed in the oral cavity when, for example, eliminating bacteria in the oral cavity, curing resin used in dental caries treatment, and activating titania. In addition, internal organs such as the stomach and the esophagus can be observed with an endoscope or the like through the oral cavity. In the past, light irradiation in the oral cavity as described above has been carried out using a light-emitting device that requires an electric circuit or wiring.

### Disclosure of the Invention

It is an object of the present invention to provide a means whereby the oral cavity can easily and safely be irradiated with light without using a light-emitting device that requires an electric circuit or wiring.

As a result of intensive studies to solve the above problem, the inventors of the present invention have found that the oral cavity can be irradiated with light by applying illuminant or fluorescence substance to a mouthpiece and introducing the mouthpiece comprising illuminant or fluorescence substance into the oral cavity. This has led to the completion of the present invention.

That is, according to the present invention, a light-emitting device for medical use that is composed of resin comprising illuminant or fluorescence substance is provided.

Preferably, the device of the present invention is a light-emitting device for the oral cavity that is composed of a mouthpiece comprising illuminant or fluorescence substance.

Preferably, the mouthpiece comprising illuminant or fluorescence substance is obtained by mixing resin with illuminant or fluorescence substance and molding the resulting resin.

Preferably, the mouthpiece comprising illuminant or fluorescence substance is obtained by applying illuminant or fluorescence substance to a mouthpiece.

Preferably, illuminant or fluorescence substance is removably applied to the mouthpiece.

Preferably, the illuminant or fluorescence substance is a bioluminescent/chemiluminescent agent or a light-storing fluorescence substance, respectively.

Preferably, the bioluminescent/chemiluminescent agent is luminol, lucigenin, hyperoxalic acid ester, or aequorin.

Preferably, the light-storing fluorescence substance is strontium aluminum borate, europium, magnesium, or yttrium oxide-sulfide activated with titanium.

### Brief Description of the Drawings

Fig. 1 shows a schematic diagram of a mouthpiece in one embodiment of the present invention. In the figure, reference numerals 1, 2, 3, 4, and 5 denote a mouthpiece, a groove, a sidewall, a sidewall, and an edge, respectively.
Fig. 2 shows results of decolorization of hematoporphyrin using a variety of illuminant.

### Best Mode for Carrying Out the Invention

Hereafter, the method and embodiments for carrying out the present invention will be described.

A light-emitting device for the oral cavity of the present invention is composed of a mouthpiece comprising illuminant or fluorescence substance. In the present invention, practical use of a light-emitting device for dental use in which illuminant or fluorescence substance, for example, are incorporated into a mouthpiece, is achieved. The present invention is characterized in that it has been discovered and realized that bioluminescence/chemiluminescence is available in the bio-related field, and particularly in the medical field, without the requirement of an electric circuit or wiring.

More specifically, the present invention relates to a light-emitting device used for teeth bleaching, bacteria elimination in the oral cavity, and resin curing. The device is composed of a mouthpiece that has been made by mixing illuminant or fluorescence substance with resin and molding the mixture, or has been made by applying illuminant or fluorescence substance to a mouthpiece.

Preferably, the mouthpiece used in the present invention is formed into a shape suitable for the application of the mouthpiece to the tooth form of a person or to a part thereof.

One feature of the present invention is that a mouthpiece is used for retention of illuminant or fluorescence substance. Heretofore, mouthpieces have mainly been used for the purpose of protection of the oral cavity during exercise, prevention of bruxism during sleep, administration of toothpaste or a bleaching agent (when administering toothpaste or a bleaching agent, the mouthpiece is worn for a short time, about several minutes, in general), and the like. The inventors of the present invention are the first to have found that a mouthpiece can be used for the purpose of light irradiation in the oral cavity.

That is, the findings of the inventors of the present invention are novel and creative in the sense that irradiation of light generated from illuminant or fluorescence substance, which have been contained in or applied to a mouthpiece, causes teeth bleaching, bacteria elimination in the oral cavity, or resin curing, each of which results in realization of expected effects.

The term "mouthpiece" used herein indicates a device that can cover teeth and/or the gum when such mouthpiece is inserted into the oral cavity. The term imparts a concept that encompasses a dental tray, a mouthguard, a night form preguard, and the like, which have been conventionally known. Such mouthpiece is not particularly limited in terms of, for example, shape, size, or material thereof, as long as it satisfies the requirements described herein. Particularly preferably, the mouthpiece does not disturb oral functions such as respiration and conversation, and is easily removable. However, the mouthpiece does not easily fall off after being applied, and can be used without causing discomfort.

The structure of the mouthpiece can be divided into a body part and a tooth form part (which comes into contact with teeth and the gum) based on function. A mouthpiece is classified as corresponding to one of the following three types, depending on the person responsible for molding the body part and the tooth form part: (1) stock type (where the body part and the tooth form part are molded by a manufacturer); (2) mouth-formed type (where the body part and the tooth form part are molded by a user); and (3) custom-made type (where the body part and the tooth form part are molded by a professional technician such as a dentist or a dental technician). A mouthpiece of the present invention may be classified as corresponding to any one of the above three types. Further, the type of a mouthpiece of the present invention may be an adequate combination of the above three types, such as a combination of a stock or custom-made type and a mouth-formed type in a manner such that the body part is molded by a manufacturer or a professional technician, and then only the tooth form part is molded by a user.

Preferably, the mouthpiece used in the present invention can be formed into a shape suitable for the application of the mouthpiece to the tooth form of a person or to a part thereof. The phrase "a shape suitable for application" indicates that the mouthpiece merely has to have a shape suitable for the application of the mouthpiece to the tooth form of the average person (for instance, when an adult is subjected to a treatment, a mouthpiece for adults, the size and shape of which correspond to the tooth form of the average adult, can be produced, and when a child is subjected to a treatment, a mouthpiece for children, the size and shape of which correspond to the tooth form of the average child, can be produced). Thus, a mouthpiece does not necessarily have an original shape that perfectly corresponds to the tooth form of a specific individual. For instance, in the case of a mouth-formed type mouthpiece, a mouthpiece formed with thermoplastic resin, which has a shape suitable for the application of the mouthpiece to the tooth form of the average person, is made. Immediately before use for a specific patient, resin of the mouthpiece is plasticized by heating (e.g., by soaking it in hot water) such that the mouthpiece is formed into a shape corresponding to the tooth form of the patient. Then, illuminant or fluorescence substance are applied to the mouthpiece, and then the mouthpiece can be applied to the patient.

Meanwhile, in the case of a stock type or custom-made type mouthpiece, a mouthpiece having a shape corresponding to the tooth form of a patient is originally made. Thus, such mouthpiece can be used without plasticization. In such case, the material to be used for the mouthpiece may be thermoplastic resin or the like.

The phrase "to the tooth form...or to a part thereof" used herein indicates that the scope of the present invention includes not only a mouthpiece that is applied to the entire tooth form of the upper or lower jaw, but also a mouthpiece that is applied to a part of such a tooth form. That is, the mouthpiece used in the present invention may fit a part of the tooth form such as teeth on the left side of the upper or lower jaw, or front teeth. Since a mouthpiece is applied to a patient subjected to treatment in a manner such that the shape of such mouthpiece corresponds to the tooth form of the patient, even a mouthpiece for a part of the tooth form can be securely applied. However, when a mouthpiece for a part of the tooth form is used, a holding means may be used so that a mouthpiece applied to teeth is not easily removed or displaced. Such holding means may be any means whereby a patient never or rarely feels discomfort such as pain, and whereby the mouthpiece can easily be removed after treatment; however, the holding means is not particularly limited thereto. Examples of such holding means include fastenings formed with metals or plastics, adhesives, and adhesive tapes.

Materials used for producing the mouthpiece of the present invention are not particularly limited, and any materials known in the field can be used. Preferably, such materials are excellent in terms of durability, formability, and shock absorption, and are not (or are only slightly) biohazardous, cost effective, and easily available. The material used may be any hard material (such as metal or rigid resin) or soft material (such as soft resin). In addition, the material may be transparent, translucent, or colored. The material can be adequately selected according to the type of a mouthpiece produced (stock type, mouth-formed type, custom-made type, or a combination thereof).

For instance, examples of a material having a relatively high softening point, which is used for a stock type or custom-made type mouthpiece, include vulcanized rubber and elastic plastic. More specifically, a rubber elastic body such as silicon rubber, ethylene-vinyl acetate (EVA) or polyolefin can be used. Preferably, thermosetting resin can be used.

In addition, as an example of thermoplastic resin having a low softening point, which is used for a mouth-formed type, a hot-melt material mainly consisting of a plastic elastic body, which becomes softened in a hot water between approximately 60°C to 100°C, can be used. Examples of such hot-melt material include ethylene-vinyl acetate (EVA).

A mouthpiece (mouth-formed type) in which the body part and the tooth form part are formed with thermoplastic resin, is preferable in view of the ability of a patient to easily mold a mouthpiece himself or herself immediately before use in a manner such that the shape of the mouthpiece corresponds to his or her tooth form. Meanwhile, in the practice of dental medicine, preferably, a mouthpiece (custom-made type) formed with thermosetting resin is used.

The mouthpiece used in the present invention may be a disposable type mouthpiece, which is discarded after a single use, or a reusable type mouthpiece, which is collected after use and is reused following adequate washing or the like. Based on the adequate selection of the material used, or the like, either a disposable type or reusable type mouthpiece can be produced according to need.

The mouthpiece used in the present invention comprises illuminant or fluorescence substance. In the present invention, for instance, a mouthpiece obtained by mixing illuminant with resin and molding the resulting resin or a mouthpiece obtained by applying illuminant or fluorescence substance to the mouthpiece can be used. As an example of the present invention, illuminant or fluorescence substance can be removably applied to a mouthpiece. Specifically, illuminant or fluorescence substance can be incorporated into the lower layer or the sidewalls (facing the lip side and the cheek side in the oral cavity) of a mouthpiece. Alternatively, the mouthpiece may have a detachable double-layer structure so that illuminant or fluorescence substance are removably applied between the upper layer and the lower layer. In addition, it is also possible to apply a paint containing illuminant or fluorescence substance to the entire mouthpiece or to a part thereof, print a mouthpiece with such paint, or apply a fluorescent seal to the mouthpiece.

In the present invention, the illuminant or fluorescence substance contained in or applied to the mouthpiece are not particularly limited, on the condition that they can emit light. Examples thereof include bioluminescent/chemiluminescent agents or light-storing fluorescence substance. Such bioluminescent/chemiluminescent agents that can be used are luminol, lucigenin, hyperoxalic acid ester (hyperoxalester), aequorin, or the like. Examples of such light-storing fluorescence substance that can be used include strontium aluminum borate, europium, magnesium, yttrium oxide-sulfide activated with titanium, and the like.

The amounts of illuminant or fluorescence substance contained in or applied to a mouthpiece can adequately be selected in accordance with types of illuminant or fluorescence substance, the desired strength of light irradiation, the patients, the duration of the wearing of the mouthpiece, or the like. The amounts of illuminant or fluorescence substance contained in a mouthpiece are preferably 3% to 80% by weight, and more preferably 15% to 50% by weight.

At least one type of illuminant or fluorescence substance is contained in or applied to a mouthpiece used in the present invention. Further, any supplemental drugs, including the following (1) to (3), may be used in combination: (1) dental treatment drugs; (2) disinfectants for affected areas; and (3) absorbents used for absorption of body fluids such as saliva, blood and pus. In addition, the mouthpiece used in the present invention is kept in a mouth for a certain period of time for dental disease treatment. During such period, body fluids such as saliva, blood and pus may be produced or effused in lesions. When such body fluids may negatively influence dental disease treatment, or when such body fluids undesirably cause a patient to feel discomfort, it is preferable that an absorbent be retained on a mouthpiece such that the body fluids are absorbed thereinto.

The duration for which the mouthpiece used in the present invention is worn is not particularly limited. However, preferably, the duration is to an extent such that a patient does not experience considerable inconvenience in his or her daily life, and at the same time, to an extent such that sufficient treatment effects can be achieved. As a specific example, the duration may range from minutes to hours (e.g., 1 minute to 10 hours), tens of hours (e.g., 10 hours to 24 hours), days (e.g., a day to 9 days), or tens of days (e.g., 10 days to 30 days or more). Thus, the duration can be freely determined.

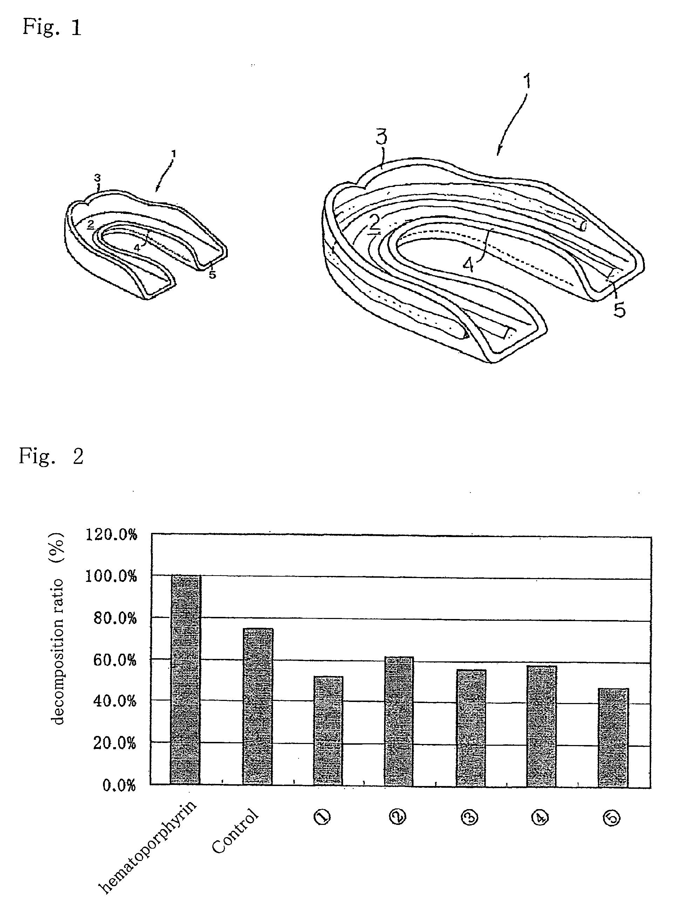

Hereafter, the shape of a mouthpiece and usage thereof will be described more specifically with reference to Fig. 1. Note that the mouthpiece described in fig. 1 merely indicates one embodiment of the present invention, and the mouthpiece is not limited to the shape described in Fig. 1.

Preferably, mouthpiece 1 is formed with thermosetting resin or thermoplastic resin, in which groove 2 is formed along with the tooth form. On the one side of groove 2, sidewall 3 that faces the jaw side and the lip side in the oral cavity is formed, and on the other side thereof, sidewall 4 that faces the palate side in the oral cavity is formed. In addition, mouthpiece 1 can be produced by adequately adjusting the heights and lengths extending to edge 5, of sidewalls 3 and 4, depending on the lengths of teeth and the gum of a user. Or, mouthpiece 1 can be cut using scissors or the like immediately before use.

When mouthpiece 1 is formed with thermoplastic resin, a container filled with hot water is previously prepared and mouthpiece 1 is soaked with such hot water. After mouthpiece 1 becomes plasticized, the tooth form is made on the mouthpiece by applying it to teeth of the upper or lower jaw and the gum of a patient and having the patient bite it softly or press it with fingers.

Then, the mouthpiece on which the tooth form has been made is removed from the mouth, an adequate amount of illuminant or fluorescence substance and, if necessary, a desired supplemental drug are applied to the inside of groove 2 (that is, where teeth and/or the gum of the patient come into contact with the mouthpiece), and then the mouthpiece is applied again to the teeth and the gum. In addition, when using the mouthpiece of the present invention, it is preferable that toothbrushing is previously carried out using a toothbrush such that mouthpiece 1 can tightly come into contact with teeth and the gum.

After the elapse of a given time for wearing mouthpiece 1, the mouthpiece is removed from the jaw so that the treatment can be terminated. After use, mouthpiece 1 (in the case of reusable type) is cleanly washed with warm water and a wash or the like, and thus it can be stored before reuse.

The present invention is hereafter described more specifically with reference to the following examples, although the scope of the present invention is not limited thereto.

### Examples

### Example 1: Mouthpieces comprising a variety of illuminant and/or fluorescence substance

Plastic was mixed with illuminant and/or fluorescence substance listed below using a blend loader. Each of the resulting melted fluorescent resin materials was molded into a plate or mouthpiece shape.
(1) ethylene-vinyl acetate copolymer: 75%; and strontium aluminum borate: 25%
(2) polycarbonate: 79.9%; strontium aluminum borate: 20.0%; and blue dye: 0.1%
(3) polyethylene: 69.8%; strontium aluminum borate: 30.0%; and red dye: 0.2%
(4) PET: 70%; and strontium aluminum borate: 30.0%
(5) polypropylene: 75%; strontium aluminum borate: 15.0%; and EVAL: 10%
(6) cyclic polyolefin: 65%; and strontium aluminum borate: 35.0%
(7) polyether sulphone: 70%; and strontium aluminum borate: 30%
(8) vinyl chloride: 97%; and strontium aluminum borate: 3%
(9) chemically polymerized resin: 55%; and strontium aluminum borate: 45.0%
(10) photopolymerized resin: 65%; and strontium aluminum borate: 35.0% (where the photopolymerized resin used herein is described in JP Patent Publication (Kokai) No. 10-1408 (1998) and is composed of: sodium methacrylate: 2%; methacrylate: 40%; triethylene glycol dimethacrylate: 52%; camphorquinone: 2%; dimethylaminoethyl methacrylate: 2%; and hydroxyethyl methacrylate: 2%)
(11) silicon gum (KE1988): 50%; acrylic resin (GC Acron): 10%; and strontium aluminum borate: 40%
(12) PFA shrinkable tube: 70%; and strontium aluminum borate: 30%

### Examples 2: Mouthpieces having a variety of illuminant and/or fluorescence substance applied thereto

Tooth-form-shaped molded products were used, to which the following materials were incorporated, injected, applied, or attached: the light-storing material (e.g., strontium aluminum borate) of Example 1; Ultra Glow (Nichia), which have been commercially available as party goods, etc.; Brightex (Tokyo Shiryo Insho KK); Keprus (Next I Co., Ltd.); PG; PB light-storing materials (α-FLASH) (LTI Corporation); bioluminescent/chemiluminescent agents such as illuminant using hydrogen peroxide, urea peroxide, hypoxanthine, and superoxide (which cause, for example, luminol, luciferin, lucigenin, MPEC, hyperoxalic acid ester (hyperoxalester), allyl oxalate, and trichlorophenyl oxalate luminescences) and illuminant using calcium (which cause an aequorin luminescence); and Cyalume (cyanamide; Omniglow) or Lumica light, which have been commercially available as party goods, etc.
(1) A sheet made of ethylene-vinyl acetate copolymer and the resin material made in (1) of Example 1 were made to overlap with each other, and were molded into the shape of a tooth form.
(2) A sheet made of ethylene-vinyl acetate copolymer and the resin material made in (12) of Example 1 were made to overlap with each other, and were molded into the shape of a tooth form.
(3) A Lumica light was incorporated into a slit made on a commercially available night guard.
(4) A luminol solution was placed inside a plastic tube, one end of which was sealed. The other side of the tube was covered with a rubber cap. A potassium ferricyanide solution was injected into the tube via the rubber cap, following which mixing took place. Thus, light was emitted from the tube. The light-emitting plastic tube was incorporated into a slit made on a commercially available night guard.
(5) A hyperoxalic acid ester (hyperoxalester)-anthracene solution was placed inside a glass tube, one end of which was sealed. The other side of the tube was covered with a rubber cap. A hydrogen peroxide-sodium salicylate solution was injected into the tube via the rubber cap, following which mixing took place. Thus, light was emitted from the tube. The light-emitting glass tube was incorporated into a slit made on a commercially available night guard.

### Examples 3:

A hematoporphyrin solution (2 ml) adjusted to 100 ppm was mixed with 1 g of hydroxyapatite. A colored substance was obtained. To the colored substance, approximately 2 ml of a hydrogen peroxide solution was added, following which mixing took place. The resultant was applied to resin samples. Then, each sample was covered with a small box. 15 minutes thereafter, the colored substance on each sample was collected and subjected to centrifugal separation. Each of the obtained supernatants was subjected to determination using a spectrophotometer. The results are shown in Fig. 2.

In Fig. 2, results of following cases are shown: control: a petri dish; (1): a sample in a petri dish to the bottom side of which Lumica light was attached; (2): a sample in a petri dish to the bottom side of which Brightex was attached; (3): a sample in a petri dish to the bottom side of which Ultra Glow was attached; (4): a sample in a petri dish to the bottom side of which Keprus was attached; and (5) a sample obtained by mixing ethylene-vinyl acetate copolymer with strontium aluminum borate and molding the mixture into a plate shape.

### Industrial Applicability

According to the present invention, it becomes possible to provide a means whereby the oral cavity can easily be irradiated with light without using a light-emitting device that requires an electric circuit or wiring.

## Claims

1. A light-emitting device for medical use that is composed of resin comprising illuminant or fluorescence substance.

2. The device according to claim 1, which is a light-emitting device for the oral cavity that is composed of a mouthpiece comprising illuminant or fluorescence substance.

3. The light-emitting device for the oral cavity according to claim 2, wherein the mouthpiece comprising illuminant or fluorescence substance is obtained by mixing resin with illuminant or fluorescence substance and molding the resulting resin.

4. The light-emitting device for the oral cavity according to claim 2, wherein the mouthpiece comprising illuminant or fluorescence substance is obtained by applying illuminant or fluorescence substance to a mouthpiece.

5. The light-emitting device for the oral cavity according to claim 4, wherein the illuminant or fluorescence substance is removably applied to the mouthpiece.

6. The device according to any of claims 1 to 5, wherein the illuminant or fluorescence substance is a bioluminescent/chemiluminescent agent or a light-storing fluorescence substance.

7. The device according to claim 6, whertein the bioluminescent/chemiluminescent agent is luminol, lucigenin, hyperoxalic acid ester, or aequorin.

8. The device according to claim 6, whertein the light-storing fluorescence substance is strontium aluminum borate, europium, magnesium, or yttrium oxide-sulfide activated with titanium.
